# EUROPÄISCHE PATENTANMELDUNG

(11) **EP 1 300 139 A2**
(43) Veröffentlichungstag der Anmeldung: **09.04.2003**
(21) Anmeldenummer: 03000323.0
(22) Anmeldetag: 13.10.1998
(51) Int. Cl.: A61K 9/16, A61K 9/51, A61K 9/00

(54) **Arzneistoffträgerpartikel für die gewebspezifische Arzneistoffapplikation**

(30) Priorität: 17.10.1997 DE 19745950
(62) Teilanmeldung aus: 98955425.8
(71) Anmelder: DDS DRUG DELIVERY SERVICE GESELLSCHAFT ZUR FÖRDERUNG DER FORSCHUNG IN PHARMAZEUTISCHER TECHNOLOGIE UND BIOPHARMAZIE MBH, 24119 Kronshagen (DE)
(72) Erfinder: Die Erfindernennung liegt noch nicht vor
(74) Vertreter: UEXKÜLL & STOLBERG

(57) **Zusammenfassung**

Die Anmeldung betrifft Arzneistoffträgerpartikel, die für die Gewebespezifische Arzneistoffappplikation, speziell zum zentralen Nervensystem (ZNS), geeignet sind und in Wirkstoff-beladener oder Wirkstoff-freier Form vorliegen, wobei an die Partikeloberfläche mindestens ein Erkennungsprotein gebunden ist oder die Partikeloberfläche so modifiziert ist, daß bei Kontakt mit einem Erkennungsprotein dieses daran gebunden wird.

## Beschreibung

Die Erfindung betrifft Arzneistoffträgerpartikel, die für die Gewebespezifische Arzneistoffappplikation, speziell zum zentralen Nervensystem (ZNS), geeignet sind.

Die Therapie von Erkrankungen des ZNS wird durch die Blut-Hirn-Schranke, einer der wichtigsten und undurchlässigsten physiologischen Barrieren im Organismus, erschwert. Als morphologisches Substrat der Blut-Hirn-Schranke wird in erster Linie das Gefäßendothel der Hirnkapillaren angesehen, da die Interzellulärspalten zwischen den Endothelzellen durch dichte Zell-Zell-Verbindungen ("tight junctions") überbrückt sind. Die Endothelzellen sind darüber hinaus von einer lückenlosen Basalmembran umschlossen. Typisch für das Gewebe sind die fehlende Fenestrierung, die Abwesenheit von Poren und eine geringe pinozytotische Aktivität. Hinzu kommt, daß die Blutgefäße im Bereich des ZNS von einer dicht anliegenden Schicht von Gliazellen eingehüllt sind (Thews, G., Mutschler, E., Vaupel, P., *Anatomie, Physiologie und Pathophysiologie des Menschen,* 3. Aufl., Wissenschaftliche Verlagsgesellschaft, Stuttgart, 1989; Borchard, G., in: Müller, R. H., Hildebrand, G. (Hrsg.), *Pharmazeutische Technologie: Moderne Arzneiformen.* Wissenschaftliche Verlagsgesellschaft, Stuttgart, 1997, 291-296). Das Gehirn kann daher vom Blut aus in der Regel nur von lipophilen Pharmaka mit einem geringen Molekulargewicht (MG < 500) erreicht werden (Pardridge, W. M., *J. Control. Rel.,* 39, 281-286, 1996).

Für sehr viele Wirkstoffe wie z.B. Peptide, Proteine und Oligonukleotide als mögliche Therapeutika für Krankheiten des ZNS ist die Blut-Hirn-Schranke normalerweise nicht permeabel.

Nach Pardridge (*J. Control. Rel.,* 39, 281-286, 1996) können die Strategien für einen Arzneistofftransport ins Gehirn eingeteilt werden in
a) invasive,
b) pharmakologische und
c) physiologische Verfahren.

Mit invasiven Techniken kann die Blut-Hirn-Schranke physikalisch umgangen werden, indem z.B. ein Arzneistoffträgersystem ins Gehirn implantiert wird (Domb, A. J., Ringel, I., in: Flanagan, T. R., Emerich, D. F., Winn, S. R. (Hrsg.), *Providing Pharmacological Access to the Brain.* Academic Press, Inc., New York, 1994, 169-187; Friden, P. M., *J. Control. Rel.,* 46, 117-128, 1996). Nachteilig bei diesen Techniken ist, daß sie mit einem chirurgischen Eingriff verbunden sind und sich deshalb nicht als gängige Behandlungsmethode etabliert haben.

Die pharmakologischen Strategien für einen Arzneistofftransport durch die Blut-Hirn-Schranke umfassen Maßnahmen zur Erhöhung der Lipophilie von Arzneistoffen (Chekhonin, V. P., Kabanov, A. V., Zhirkov, Y. A., Morozov, G. V., *FEBS Lett.,* 287, 149-152, 1991). Nachteile dieser Verfahren sind, daß "new drug entities" entstehen, für die umfangreiche kostenintensive toxikologische Untersuchungen gemacht werden müssen, daß diese Verfahren nur für relativ kleine Moleküle praktikabel sind und daß sie eine geringe Effizienz besitzt (Friden, P. M., *J. Control. Rel.,* 46, 117-128, 1996; Pardridge, W. M., *J. Control. Rel.,* 39, 281-286, 1996).

Physiologische Strategien für einen Arzneistofftransport ins Gehirn basieren auf der Kenntnis spezieller aktiver spezifischer Transportmechanismen an der Blut-Hirn-Schranke z.B. für Nährstoffe (u.a. Glucose und Aminosäuren), Peptide oder Proteine (Pardridge, W. M., *Peptide Drug Delivery to the Brain,* Raven Press, New York, 1991; Pardridge, W. M., *J. Control. Rel.,* 39, 281-286, 1996; Friden, P. M., *J. Control. Rel.,* 46, 117-128, 1996). Ein Beispiel ist L-Dopa als Vorstufe des Neurotransmitters Dopamin, welcher die Blut-Hirn-Schranke nicht zu überwinden vermag. L-Dopa hingegen wird durch einen aktiven Transportweg für neutrale Aminosäuren ("Neutrale Aminosäuren-Carrier") durch die Blut-Hirn-Schranke in die Zellen des Gehirns transportiert, wo die eigentliche Wirkform Dopamin gebildet wird (Mutschler, *E., Arzneimittelwirkungen, Lehrbuch der Pharmakologie und Toxikologie,* 7. Aufl., Wissenschaftliche Verlagsgesellschaft, Stuttgart, 1996; Borchard, G., in: Müller, R. H., Hildebrand, G. (Hrsg.), *Pharmazeutische Technologie: Moderne Arzneiformen.* Wissenschaftliche Verlagsgesellschaft, Stuttgart, 1997, 291-296). Aber auch dieser Ansatz hat sich aufgrund folgender Nachteile nicht auf breiter Ebene durchgesetzt:
1. die aktiven Transportmechanismen sind sehr Substrat-spezifisch, d.h., nur wenige Arzneistoffe mit großer Ähnlichkeit zum Substrat werden transportiert, was die Einsatzfähigkeit dieser Strategie sehr limitiert.
2. Konjugate aus natürlichem Substrat und Arzneistoff werden wegen der großen Spezifität des Transporters (chemische Struktur und dreidimensionale Struktur und Größe des zu transportierenden Substrats) nicht oder wenig effizient transportiert.

Ein weiterer Ansatz für die gewebespezifische Arzneistoffapplikation, z.B. ins ZNS, ist die Einarbeitung von Arzneistoffen in partikuläre Arzneistoffträger wie Nanopartikel, Mikropartikel, Emulsionen und Liposomen sowie Verarbeitung zu partikulären Arzneiformen wie Hydrosole, Nanokristalle und Nanosuspensionen. Generell ist für intravenös injizierte Partikel die Überwindung von Endothelien aufgrund ihrer Größe (in der Regel » 30 nm) noch schwieriger als für Arzneistoffmoleküle (Größe im Ångström-Bereich). So wird beispielsweise für Liposomen generell eine sehr begrenzte Fähigkeit zur Penetration durch die Blut-Hirn-Schranke beschrieben (Gennuso, R., Spigelman, M. K., Chinol, M., Zappulla, R. A., Nieves, J., Vallabhajosula, S., Paciucci, P. A., Goldsmith, S. J., Holland, J. F., *Cancer Invest.*, 11, 118-128, 1993; Boado, R. J., *Adv. Drug Deliv. Rev.,* 15, 73-107, 1995; Boado, R. J., *Proceed. Intern. Symp. Control. Rel. Bioact. Mater.,* 24, 223-224, 1997; Pardridge, W. M., *J. Control. Rel.,* 39, 281-286, 1996).

Einen ersten Erfolg hinsichtlich der Applikation eines Arzneistoffs zum ZNS mit partikulären Trägern publizierten Alyautdin et al. (Alyautdin, R. N., Gothier, D., Petrov, V. E., Kharkevich, D. A., Kreuter, J., *Eur. J. Pharm. Biopharm.,* 41, 44-48, 1995). Sie demonstrierten für i.v. applizierte Poiybutylcyanoacrylat(PBCA)-Nanopartikel, auf deren Oberfläche die analgetisch wirksame Substanz Dalargin durch Adsorption gebunden wurde, einen dosisabhängigen analgetischen Effekt im "tail-flick-Test" an Mäusen. Das Hexapeptid Dalargin (Tyr-D-Ala-Gly-Phe-Leu-Arg) ist ein Leu-Enkephalin-Analogon und besitzt als Opioid-Rezeptor-Agonist eine zentral analgetische Wirkung. Dalargin kann normalerweise die Blut-Hirn-Schranke nicht überwinden.

Eine i.v. Applikation von Dalargin führt trotz der Stabilität im Blut auch in hoher Dosierung (20 mg/kg) zu keinem analgetischen Effekt (Kalenikova, E. I., Dmitrieva, O. F., Korobov, N. N., Zhukova, S. V., Tischenko, V. A., *Vopr. Med. Khim*., 34, 75-83, 1988).

In einer anderen Studie konnte im Rattenmodell nach intravenöser Injektion von oberflächenmodifizierten Polymethylmethacrylat-(PMMA)-Nanopartikeln eine Anreicherung der Partikel im Gehirnbereich detektiert werden (Tröster, S. D., Müller, U., Kreuter, *J., Int. J. Pharm.,* 61, 85-100, 1990). Die Autoren hielten es aber für ausgeschlossen, daß die Partikel in Gehirnzellen aufgenommen werden, was eine Applikation von Arzneistoff ins Gehirn ausschließt.

Nachteilig ist, daß das von Alyautdin et al. (*Eur. J. Pharm. Biopharm.,* 41, 44-48, 1995) und von Schröder und Sabel *(Brain Res.,* 710, 121-124, 1996) berichtete Phänomen nicht für eine gezielte und kontrollierte Arzneistoffapplikation benutzt werden kann. Der Mechanismus ist nicht bekannt. Es bleibt nur die "Trial-und-Error-Vorgehensweise", um zu detektieren, ob eine Zumischung eines Tensids zu einem partikulären Träger vielleicht zufällig eine Anreicherung im Gehirn erzeugt. Die Wahrscheinlichkeit daß es dazu kommt ist gering, da Tenside oft in Partikelpräparationen eingesetzt wurden (Couvreur, P., Dubernet, C., Puisieux, F., Eur. *J. Pharm. Blopharm*., 41, 2-13, 1995) und bisher die obigen Berichte die ersten Daten über eine Aufnahme eines Arzneistoffes in das Gehirn sind.

Für einen Arzneistofftransport spezifisch in das gewünschte Zielgewebe, insbesondere auch ins Gehirn, wäre eine Arzneiform optimal, die
1. die Spezifität eines Transportweges beispielsweise über Rezeptor-vermittelte Transzytose (physiologische Strategie) mit der hohen Transportkapazität partikulärer Arzneistoffträger, z.B. Liposomen, Emulsionen oder Nanopartikel, verbindet,
2. die Aufnahme des Arzneistoffes in das Gewebe - z.B. das Gehirn - über ein generell einsetzbares Erkennungsmölekül ermöglicht und
3. eine kontrollierte Befestigung des Erkennungsmoleküls an die Oberfläche von Partikeln erlaubt.

Der Vorteil von partikulären Arzneistoffträgern - im Gegensatz zu z.B. Molekülkonjugaten - liegt neben der hohen Transportkapazität in der Möglichkeit, über die Wahl der Trägermatrix (z.B. Polymer, Lipid, Phospholipid) und der Herstellungsbedingungen der Partikel viele in ihren physikochemischen Eigenschaften und ihrem Molekulargewicht unterschiedliche Arzneistoffe transportieren zu können (Borchard, G., in: Müller, R. H., Hildebrand, G. (Hrsg.), *Pharmazeutische Technologie: Moderne Arzneiformen.* Wissenschaftliche Verlagsgesellschaft, Stuttgart, 1997, 291-296).

Der Erfindung liegt daher die Aufgabe zugrunde, Arzneistoffträgerpartikel zu schaffen, die in der Lage sind, die Blut-Hirn-Schranke zu überwinden und gewünschte Arzneiwirkstoffe in das ZNS einzuführen.

Diese Aufgabe wird gemäß Anspruch 1 durch Arzneistoffträgerpartikel, in Wirkstoff-beladener oder Wirkstoff-freier Form, gelöst, bei denen an die Partikeloberfläche mindestens ein Erkennungsprotein oder zumindest der den Rezeptor erkennende Anteil davon gebunden ist.

Bevorzugte Ausführungsformen sind Gegenstand der Unteransprüche.

Als Partikelmaterial sind insbesondere Polymere, der Arzneistoff selbst (Nanosuspensionen, Hydrosole), feste Lipide, flüssige Lipide, o/w-Emulsionen, w/o/w-Emulsionen oder Phospholipidvesikel geeignet.

Arzneistoffträgerpartikel, die durch Bindung eines (auch natürlich im Blut vorkommenden) Erkennungsproteins an die Partikeloberfläche, vorzugsweise Apolipoprotein E, oder durch Bindung mehrerer Erkennungsproteine, modifiziert sind, können Arzneistoffe spezifisch zum Zielgewebe transportieren, insbesondere zum Zentralen Nervensystem (ZNS). Die Erkennungsproteine werden an die Partikeloberfläche adsorbiert, kovalent daran gebunden oder durch gezielte Modifikation der Partikeloberfläche (chemisch, physikalisch, Adsorption von die Adsorption des Erkennungsproteins vermittelnden Molekülen) präferentiell adsorbiert.

Die Adsorption der Erkennungsproteine kann vor Applikation der Arzneistoffträger erfolgen oder - nach entsprechender Modifikation der Oberfläche der Arzneistoffträger - auch in vivo im Körper. Im Körper vorhandene, physiologische Erkennungsproteine, insbesondere Apolipoprotein E, adsorbieren präferentiell auf applizierten Arzneistoffträgern, insbesondere auf mit speziellen Tensiden oder Polymeren modifizierten Trägern, die beispielsweise durch intravenöse Applikation in den Körper eingebracht worden sein können.

Das Erkennungsprotein kann durch unspezifische oder spezifische Adsorption an die Oberfläche der Partikel gebunden sein.

Ferner kann das Erkennungsprotein kovalent an die Oberfläche der Partikel gebunden sein. Dabei wird worzugsweise eine Bindung an Partikel mit reaktiven Oberflächengruppen, insbesondere Epoxyoder Aldehydgruppen, oder nach Aktivierung der Partikeloberfläche mit Aktivatoren, insbesondere Carbodiimid, N-Ethoxycarbonyl-2-ethoxy-1,2-dihydrochinolin, Glutardialdehyd, Bromzyan, meta-Perjodat (Na-Salz oder K-Salz), Tosylchlorid und Chlorameisensäureester bewirkt. Dabei kann die Bindung der Erkennungsproteine über ihre Aminogruppen bewirkt werden.

Außerdem kann das Erkennungsprotein durch präferentielle Adsorption an die Oberfläche der Partikel gebunden sein. Dabei kann die präferentielle Adsorption aus Proteinlösungen oder durch Kontakt der Partikel mit Plasma, Serum oder Blut erfolgen, wobei letzteres auch ex vivo oder in vivo erfolgen kann.

Die Oberfläche der Partikel kann vorzugsweise vor der präferentiellen Adsorption durch Einführung funktioneller Gruppen, insbesondere Hydroxyl-, Carboxyl-, Amino-, Hydroxyethyl, Epoxy oder Aldehydgruppen und deren Derivate chemisch modifiziert werden oder durch physikalische Behandlung mit Plasma, insbesondere plasma etching, zur Einführung von Hydroxylgruppen in den Eigenschaften verändert werden.

Ferner kann die Oberfläche durch Adsorption von Substanzen modifiziert werden, die zu einer präferentiellen Adsorption des Erkennungsproteins führen, wobei die modifizierende Substanz im Verhältnis zum Arzneistoffpartikel in einer gewichtsbezogenen Menge von 0.01 Teile bis 10 modifizierende Substanz pro 1 Teil Partikel, vorzugsweise 0,1 bis 10 Teile modifizierende Substanz pro 1 Teil Partikel, und insbesondere 1 Teil modifizierende Substanz pro 1 Teil Partikel eingesetzt wird.

Geeignete Substanzen umfassen insbesondere Tenside, speziell ethoxylierten Tenside, vorzugsweise Polyethylenglykol-Fettsäureester und Polyethylenglykol-Fettalkoholether, bevorzugt Polyetylenglykol-Sorbitanfettsäureester und Polyethylenglykol-Fettsäureglyceride, bevorzugter Tween® 20, 40, 60 und 80 oder Cremophor® EL und RH40.

Geeignet sind beispielsweise auch Polymere, insbesondere Polymere aus den Poloxameren und Poloxaminen, Cellulosen und ihren Derivaten, vorzugsweise Methylcellulose, Hydroxyethylcellulose, Hydroxypropyl-Methylcellulose, Hydroxypropylcellulose, Carboxymethylcellulose-Natrium sowie Xanthan, Polyvinylalkohol, Polyvinylpyrrolidon, Polyacrylsäuren, Polyethylenglykolen, Polyethylenglykol-enthaltenden Block-Copolymeren, Stärke und -derivaten, Dextran und -derivaten, Polyethylenimin und Gelatine.

Das Erkennungsprotein ist vorzugsweise ein im Blut natürlich vorkommendes Erkennungsprotein. Bevorzugte Erkennungsproteine sind Apolipoprotein E, Apolipoprotein A-I, A-II, A-IV, B, C-II, C-III, D, H und/oder J. Das Erkennungsprotein ist vorzugsweise Apolipoprotein E, das in Kombination mit einem oder mehreren anderen Erkennungsproteinen vorliegen kann, insbesondere mit Apolipoprotein A-I, A-II, A-IV, B, C-II, C-III, D, H und/oder J, und/oder mit Albumin.

Apo C-II, Apo C-III, Apo A-IV, Apo-E werden vorzugsweise einzeln oder in Kombinationen von 2 oder 3 oder 4 Apolipoproteinen eingesetzt.

Das Erkennungsprotein wird, bezogen auf den Arzneistoffträger, im allgemeinen in einer Menge von 0,001 bis 40 Gew.-%, insbesondere von 0,01 bis 30 Gew.-% und bevorzugt von 0,1 bis 15 Gew.-% eingesetzt.

Für Apolipoprotein (Apo) E existieren verschiedene Rezeptoren, die zu der Familie der LDL-Rezeptoren gehören (Schneider, W. J., Nimpf, J., *Curr. Opin. Lipid., 4,* 205-209, 1993).

ApoE-Rezeptoren finden sich ebenfalls an der Blut-Hirn-Schranke. Im Liquor cerebrospinalis wird die Aufnahme von Lipoproteinen über zelluläre LDL-Rezeptoren nach bisherigen Erkenntnissen ausschließlich von ApoE vermittelt. ApoE wird vorwiegend in den Astrozyten synthetisiert und abgesondert. Diese Zellen exprimieren auch LDL-Rezeptoren (Boyles, J. K., Pitas, R. E., Wilson, E., Mahley, R. W., Taylor, J. M., *J. Clin. Invest.,* 76, 1501-1513, 1985; Weisgraber, K. H., Roses, A. D., Strittmatter, J., *Curr. Opin. Lipid*., 5, 110-116, 1994).

Zusammenfassend kann festgestellt werden, daß in Bereichen des Gehirns und der Blut-Hirn-Schranke verschiedene Rezeptor-Systeme existieren, die einen Transport von ApoE bzw. ApoE-haltigen Partikeln ins Gehirn ermöglichen könnten.

Um die Rolle des Proteins nachzuweisen, war zu zeigen, daß eine Anreicherung eines Partikels in einem Gewebe erfolgt, nachdem Bindung des für die Anreicherung als ursächlich angesehenen Apolipoproteins E an die Oberfläche des Partikels vorgenommen wurde.

Für den Nachweis der Funktion von ApoE wurden Partikel eingesetzt, an deren Oberfläche ApoE aus Plasma nicht adsorbiert.

Verwendet wurden daher unmodifizierte Polybutylcyanoacrylat-(PBCA)-Partikel aus Beispiel 1, für welche die Abwesenheit von ApoE auf der Oberfläche nach Inkubation mit Plasma nachgewiesen wurde. Inkubation der Partikel mit einer Lösung von ApoE führte zu einer Adsorption auf der Oberfläche (Beispiel 2). Es ist somit möglich ApoE durch einen einfachen Adsorptionsprozeß an die Oberflächen partikulärer Träger zu binden.

### Überraschend ist mit ApoE somit ein Protein identifiziert worden, das gleichzeitig

a) in relativ großer Menge auf gewebespezifischen Partikeln vorhanden war,
b) bei dessen Anwesenheit eine ZNS-Wirksamkeit auftrat (Beispiel 1),
c) bei dessen Abwesenheit eine ZNS-Wirksamkeit fehlte (Beispiel 4),
d) und mit dem aufgrund der in der Literatur beschriebenen Rezeptoren ein Erkennungs- und Vermittlungseffekt auch theoretisch möglich ist.

Die richtige Konformation des adsorbierten Erkennungsproteins (an den Rezeptor bindendes Molekülsegment in der zum Rezeptor passenden Molekülanordnung exponiert) ist von essentieller Bedeutung für den Transport zum Zielgewebe, hier das ZNS. Unter Zulassungsgesichtspunkten für Arzneimittel (Vermeidung einer "new entity") sollte das Erkennungsprotein - wenn möglich - an die Partikel nicht kovalent gebunden werden, da dies für jede Arzneiform bei der Zulassung eine neue Toxizitätsstudie erforderlich macht. Technologisch einfacher ist die Bindung durch Adsorption an die Oberfläche.

Probleme dabei sind:
1. Adsorbiert das ApoE an die Partikeloberfläche auch ohne Gegenwart eines Tensids wie z.B. Tween® 80, welches zur automatischen Anreicherung von ApoE führt?
2. Verbleibt ApoE bei Bindung durch Adsorption nach intravenöser Injektion auf der Partikeloberfläche?
3. Ist ApoE in der richtigen Konformation adsorbiert, so daß es an das Zielgewebe binden kann und eine ZNS-Wirksamkeit vermittelt?

Besonders die mögliche Verdrängung von ApoE von der Oberfläche (siehe 2.) und die Notwendigkeit der richtigen Konformation des adsorbierten ApoE (siehe 3.) ließen es unwahrscheinlich erscheinen, daß ein Arzneistofftransport zum Gehirn erfolgen würde.

Präadsorbiertes ApoE könnte nach einer intravenösen Applikation möglicherweise von anderen Plasmaproteinen von der Partikeloberfläche verdrängt werden. Um diesen möglichen Verdrängungsprozeß zu simulieren und zu analysieren, wurden die Plasmaproteinadsorptionsmuster von PBCA-Partikeln bestimmt, an deren Oberfläche zuvor ApoE durch Adsorption gebunden worden war. Präadsorbiertes ApoE blieb auch nach nachträglicher Inkubation in Plasma auf der Oberfläche erhalten (Beispiel 3).

Der beobachtete analgetische Effekt resultiert aus einem Rezeptor-vermittelten Prozeß an der Blut-Hirn-Schranke. Daher muß auf der Oberfläche adsorbiertes ApoE in einer Konformation vorliegen, die eine Bindung an einen ApoE-Rezeptor zuläßt. Dies gilt für präadsorbiertes oder kovalent gebundenes ApoE ebenso wie für für ApoE-Moleküle, die durch Adsorption aus Plasma auf die Partikeloberfläche gelangen.

Tensidfreie PBCA-Partikel mit präadsorbiertem ApoE wurden im "tail-flick-Test" an Mäusen hinsichtlich ihres Potentials untersucht, Dalargin durch die Blut-Hirn-Schranke zu transportieren und einen analgetischen Effekt hervorzurufen. Unmodifizierte PBCA-Partikel waren nicht in der Lage, nach i.v. Applikation die Blut-Hirn-Schranke zu überwinden (Alyautdin, R. N., Gothier, D., Petrov, V. E., Kharkevich, D. A., Kreuter, J., *Eur. J. Pharm. Biopharm.,* 41, 44-48, 1995; Kreuter, J., Alyautdin, R. A., Kharkevich, D. A., Ivanov, A., *Brain Res.,* 674, 171-174, 1995; Schröder, U., Sabel, B. A., *Brain Res.,* 710, 121-124, 1996; Alyautdin, R. N., Petrov, V. E., Langer, K., Berthold, A., Kharkevich, D. A., Kreuter, J., *Pharm. Res.,* 14, 325-328, 1997). Die i.v. Applikation der PBCA-Nanopartikel mit präadsorbiertem ApoE führte jedoch zu einer analgetische Wirkung. 15 Minuten nach Injektion der Partikel wurde ein Effekt beobachtet, der 44% des maximal möglichen Effektes ausmachte (Beispiel 1). Entgegen den Erwartungen adsorbierte ApoE in der richtigen Konformation und blieb auch nach i.v. Injektion ausreichend stabil auf der Oberfläche adsorbiert, um Darlagin in das ZNS zu transportieren.

Die Tatsache, daß unmodifizierte Partikel, an die vor der i.v. Applikation ApoE gebunden wurde, einen analgetischen Effekt erzeugen, belegt die Beteiligung von ApoE an dem erhöhten Transport der Dalargin-beladenen PBCA-Partikel durch die Blut-Hirn-Schranke.

Für ApoE wird ein Modell beschrieben, nach dem das Protein aus zwei strukturell verschiedenen Domänen besteht (Weisgraber, K. H., *Adv. Prot. Chem.,* 45, 249-303, 1994; Weisgraber, K. H., Roses, A. D., Strittmatter, J., *Curr. Opin. Lipid*., 5, 110-116, 1994). Demnach besitzt ApoE eine amino-terminale globuläre (d.h. strukturell stabile) Domäne und eine carboxy-terminale labilere Domäne, die stärker zur Entfaltung ihrer Struktur neigt. Die N-terminale Domäne enthält den Teil des Proteins, der mit potentiellen Rezeptoren interagiert, die C-terminale Domäne ist für die Bindung an Lipide (z.B. in HDL) verantwortlich (Weisgraber, K. H., *Adv. Prot. Chem.,* 45, 249-303, 1994). Überraschenderweise reichte die Affinität dieser C-terminalen Domäne zu Oberflächen aus, daß
a) eine ausreichende Bindung von ApoE an die Partikeloberfläche stattfand,
b) die Adsorption ausreichend stabil war um ApoE auch bei Kontakt mit Plasmaproteinen auf der Oberfläche präsent zu halten,
c) die C-terminale Domäne und nicht die N-terminale Domäne auf der Oberfläche band und
d) dadurch die Struktur der stabileren N-terminalen Domäne und damit die Affinität zu potentiellen Rezeptoren erhalten blieb.

### Überraschend ist mit ApoE somit ein Protein identifiziert worden, das gleichzeitig

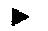 an die Oberfläche eines Partikels adsorbiert,
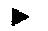 an die Oberfläche stabil gebunden bleibt und nicht vollständig durch andere Plasmaproteine verdrängt wird (z.B. nach intravenöser Injektion) und
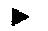 in der richtigen Konformation adsorbiert, so daß es an das Zielgewebe (in diesem Fall Blut-Hirn-Schranke und ZNS) binden kann und eine ZNS-Wirksamkeit des Arzneistoffes vermittelt.

Das oder die Erkennungsproteine können auf die Oberfläche unmodifizierter Partikel adsorbiert werden (Beispiel 2, PBCA-Partikel) oder alternativ nach vorheriger Modifikation der Oberfläche (Beispiel 2, PBCA-Partikel modifiziert durch adsorbiertes Tensid). Für die gewebespezifische anreicherung im Gehirn müssen dafür gezielt Tenside ausgewählt werden, die beispielsweise ApoE an der Oberfläche anreichern (z.B. Tween, Beispiel 2), damit in vivo eine ZNS-Wirkung auftritt (Tabelle 1). Tenside, die dies nicht bewirken, wie Poloxamer 407 (Beispiel 4) ergeben in vivo auch keine Arzneistoffwirkung im ZNS (Tabelle 1).

Die Adsorption der Erkennungsproteine kann dabei ex vivo erfolgen (z.B. aus ApoE-Lösung, Plasma, Serum, Blut), um in vivo eine ZNS-Wirkung zu erzeugen (Beispiel 1). Nach gezielter Modifikation der Partikeloberfläche zur präferentiellen Adsorption von ApoE kann dies jedoch auch in vivo nach Kontakt mit Blut erfolgen (Tabelle 1).

Bei Arzneistoffträgern mit sehr hydrophiler Oberfläche kann es sein, daß die Affinität des Erkennungsproteins nicht ausreicht, um an die Oberfläche im ausreichenden Maß zu adsorbieren. Ebenso ist es möglich, daß das zur Bindung an den Rezeptor benötigte Molekülsegment adsorbiert (im Fall von ApoE z.B. die N-terminale Domäne). In diesen Fall können die Erkennungsproteine an funktionelle Gruppen der Oberfläche kovalent gebunden werden, wobei die Bindung an die Oberfläche über Molekülsegmente erfolgt, die für die Bindung an den Rezeptor des Zielgewebes nicht benötigt werden (im Fall von ApoE z.B. die C-terminale Domäne). Beispiele 5 und 6 zeigen die kovalente Bindung eines Erkennungsproteins an die Oberfläche eines Polymernanopartikels. Alternativ zur Anbindung des gesamten Erkennungsproteins können auch Teile des Moleküls verwandt werden, die den an den Rezeptor bindnenden Molekülteil enthalten.

In nachfolgender Tabelle sind Beispiele von relevanten Aktivierungs- und Kopplungsreagentien für eine chemisch-kovalente Kopplung des Proteins an die Trägermatrix aufgelistet.

Folgende Substanzen werden für die chemische Aktivierung, als Vorstufe einer kovalenten Verknüpfung von funktionellen Gruppen eines Biomoleküls mit denen auf einem festen Träger, eingesetzt.

| Partikel | Biomolekül | Substanz * |
|---|---|---|
| -COOH | -NH₂ | Carbodiimid (wasserlöslich) (1-Ethyl-3-(3-dimcthylaminopropyl)-carbodiimid-hydrochlorid) |
| -COOH | -NH₂ | EEDQ (N-Ethuxycarbonyl-2-ethoxy-1,2-dihydrochinolin) |
| -NH₂ | -NH₂ | Glulardialdchyd |
| -OH (in aq.) | -NH₂ | Bromzyan (BrOCN) |
| -CH₂-CH₂-OH | -NH₂ | meta-Perjodat (Na/K) JO₄ |
| -OH (in org.LM) | -NH₂ | Tosylchlorid |
| -OH (in org.LM) | -NH₂ | Chlorameisensäureester |
| Sogenannte "ready to use" - Partikel mit reaktiven Oberflächengruppen benötigen keine chemische Aktivierung als Kopplungsvorstufe: | | |
| -CH(O)-CH2 Epoxy- | -NH₂ -OH -COOH - | ---- |
| -CHO Aldehyd | NH₂ | ----- |

Im allgemeinen können die Träger folgende chemische Wirkstoffgruppen enthalten:
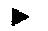 hydroxylierte Kohlenwasserstoffe
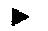 Carbonylverbindungen wie Ketone (z.B. Haloperidol), Monosaccharide, Disaccharide und Aminozucker
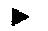 Carbonsäuren wie aliphatische Carbonsäuren, Ester aliphatischer und aromatischer Carbonsäuren, basisch substituierte Ester aliphatischer und aromatischer Carbonsäuren (z.B. Atropin, Scopolamin), Lactone (z.B. Erythromycin), Amide und Imide aliphatischer Carbonsäuren, Aminosäuren, aliphatische Aminocarbonsäuren, Peptide (z.B. Ciciosporin), Polypeptide, β-Lactamderivate, Penicilline, Cephalosporine, aromatische Carbonsäuren (z.B. Acetylsalicylsäure), Amide aromatischer Carbonsäuren, vinyloge Carbonsäuren und vinyloge Carbonsäureester
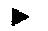 Kohlensäurederivate wie Urethane und Thiourethane, Harnstoff und Harnstoffderivate, Guanidinderivate, Hydantoine, Barbitursäurederivate und Thiobarbitursäurederivate
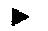 Nitroverbindungen wie aromatische Nitroverbindungen und heteroaromatische Nitroverbindungen
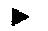 Amine wie aliphatische Amine, Aminoglykoside, Phenylalkylamine, Ephedrinderivate, Hydroxyphenylethanolamine, Adrenalinderivate, Amfetaminderivate, aromatische Amine und Derivate, quartäre Ammmoniumverbindungen
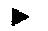 schwefelhaltige Verbindungen wie Thiole und Disulfane
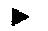 Sulfone, Sulfonsäureester und Sulfonsäureamide
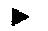 Polycarbocyclen wie Tetracycline, Steroide mit aromatischem Ring A, Steroide mit alpha,beta-ungesättigter Carbonylfunktion im Ring A und alpha Ketol-Gruppe (oder Methylketo-Gruppe) am C-17, Steroide mit einem Butenolid-Ring am C-17, Steroide mit einem Pentadienolid-Ring am C-17 und Seco-Steroide
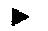 0-haltige Heterocyclen wie Chromanderivate (z.B. Cromoglicinsäure)
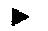 N-haltige Heterocyclen wie Pyrazolderivate (z.B. Propyphenazon, Phenylbutazon)
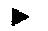 Imidazolderivate (z.B. Histamin, Pilocarpin), Pyridinderivate (z.B. Pyridoxin, Nicotinsäure), Pyrimidinderivate (z.B. Trimetoprim), Indolderivate (z.B. Indometacin), Lysergsäurederivate (z.B. Ergotamin), Yohimbanderivate, Pyrrolidinderivate, Purinderivate (z.B. Allopurinol), Xanthinderivate, 8-Hydroxychinolin-derivate, Amino-hydroxy-alkylierte Chinoline, Aminochinoline, Isochinolinderivate (z.B. Morphin, Codein), Chinazolinderivate, Benzopyridazinderivate, Pteridinderivate (z.B. Methotrexat), 1,4-Benzodiazepinderivate, tricyclische N-haltige Heterocyclen, Acridinderivate (z.B. Ethacridin) und Dibenzazepinderivate (z.B. Trimipramin)
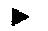 S-haltige Heterocyclen wie Thioxanthenderivate (z.B Chlorprothixen)
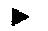 N,0- und N,S-haltige Heterocyclen wie monocyclische N,0-haltige Heterocyclen, monocyclische N,S-haltige Heterocyclen, Thiadiazinderivate, bicyclische N,S-haltige Heterocyclen, Benzothiadiazinderivate, tricyclische N,S-haltige Heterocyclen und Phenothiazinderivate
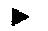 0,P,N-haltige Heterocyclen (z.B. Cyclophosphamid).

Beispiele für speziell in die Träger einzuarbeitende Arzneistoffgruppen und Arzneistoffe (als Salz, Ester, Ether oder in freier Form) sind:
Analgetika/Antirheumatika
   BTM Basen wie Morphin, Codein, Heroin, Piritamid, Diamorphin, Dihydrocodein, Hydromorphon, Hydrocodon, Pethidin, Fenpipramid, Piritramid, Clofedanol, Pentazocin, Buprenorphin, Nalbuphin, Tilidin, Fentanyl und Fentanylderivate, Levomethadon, Tramadol, Diclofenac, Ibuprofen, Indometacin, Naproxen, Piroxicam, Penicillamin, Ademetionin , Flupirtin, Acetylsalicylsäure
Antiallergika
   Pheniramin, Dimetinden, Terfenadin, Astemizol, Loratidin, Doxylamin, Meclozin, Bamipin, Clemastin
Antiasthmatika
   Terbutalin, Beclomethason, Cromoglycinsäure, Reproterol, Salbutamol, Nedocromil
Antibiotika/Chemotherapeutika
   hiervon: Rifampicin, Amoxicillin, Azlocillin, Bacampicillin, Benzylpenicillin, Amikacin, Azithromycin, Ciprofloxacin, Norfloxacin, Polypeptidantibiotika wie Colistin, Polymyxin B, Teicplanin, Vancomycin; Malariamittel wie Chinin, Halofantrin, Mefloquin, Chloroquin, Virustatika wie Ganciclovir, Foscarnet, Zidovudin, Aciclovir und andere wie Brivudin, Dapson, Fosfomycin, Fusafungin, Trimetoprim, Amphotericin
Antidota
   Mesna
Antiemetka
   Tropisetron, Scopolamin, Thiethylperazin
Antiepileptika
   Phenytoin, Mesuximid, Ethosuximid, Primidon, Phenobarbital, Valproinsäure, Carbamazepin, Clonazepam, Diazepam, Nitrazepam, Vigabatrin, Lamotrigin, Trimethadion, Sultiam
Antifibrinolytika
   Aminomethylbenzoesäure
Antihypertonika/Betarezeptorenblocker/Calciumantagonisten/ACE-Hemmer
   Bupranolol, Captopril, Fosinopril, Nitroprussidnatrium, Isradipin, Mepindolol
Antihypotonika
   Cafedrin, Dihydroergotamin
Antikoagulantien
   Heparin, Certoparin
Antimykotika
   Nystatin, Natamycin, Amphotericin B, Flucytosin, Miconazol, Fluconazol, Itraconazol, Clotrimazol, Econazol, Tioconazol, Fenticonazol, Bifonazol, Oxiconazol, Ketoconazol, Isoconazol, Tolnaftat, Amorolfin, Terbinafin
Corticoide
   Aldosteron, Fludrocortison, Betametason, Dexametason, Triamcinolon, Fluocortolon, Hydroxycortison, Prednisolon, Prednyliden, Cloprednol, Methylpredinsolon
Diagnostika
   a) radioaktive Isotope wie Te99m, In111 oder I131, kovalent gebunden an Lipide oder Lipoide oder andere Moleküle oder in Komplexen
   b) hochsubstituiertre iodhaltige Verbindungen wie z.B. Lipide
   c) Megluminamidotrizoat, Iotroxinsäure, Natriumiopodat
Diuretika
   Hydrochlorothiazid
Erythropoetin
Fibrinolytika
   Urokinase
Hämostyptika/Antihämorrhagika
   Blutgerinnungsfaktoren VIII, IX
Hypnotika, Sedativa
   Cyclobarbital, Pentobarbital, Phenobarbital, Methaqualon (BTM), Benzodiazepine (Flurazepam, Midazolam, Nitrazepam, Lormetazepam, Flunitrazepam, Triazolam, Brotizolam, Temazepam, Loprazolam), Thalidomid, Zolpidem, Zopiclon, Diphenhydramin, Doxylamin, Temazepam
Hypophysen-, Hypothalamushormone, regulatorische Peptide und ihre Hemmstoffe
   Corticotrophin, Tetracosactid, Choriongonadotropin, Urofollitropin, Urogonadotropin, Somatropin, Metergolin, Bromocriptin, Terlipressin, Desmopressin, Oxytocin, Argipressin, Ornipressin, Leuprorelin, Triptorelin, Gonadorelin, Buserelin, Nafarelin, Goselerin, Somatostatin, Quinagolid, Octreotidacetat, Lypressin
Immuntherapeutika und Zytokine
   Dimepranol-4-acetatamidobenzoat, Thymopentin, a-Interferon, β-Interferon, g-Interferon, Filgrastim, Interleukine, Azathioprin, Ciclosporin, Molgramostim, GM-CSF
Koronarmittel
   Glyceroltrinitrat, Isosorbiddinitrat, Oxyfedrin
Lebertherapeutika
   Sylimarin
Lipidsenkert
   Pravaststin, Fluvastatin
Lokalanaesthetika
   Butanilicain, Mepivacain, Bupivacain, Etidocain, Lidocain, Articain, Prilocain, Propipocain, Oxybuprocain, Tetracain, Benzocain
Migränemittel
   Proxibarbal, Lisurid, Methysergid, Dihydroergotamin, Clonidin, Ergotamin, Pizotifen, Sumatriptan
Muskelrelaxantien
   Tubocurarin, Alcuronium, Pancuronium, Vecuronium, Atracurium, Suxamethonium, Dantrolen, Baclofen, Carisoprodol, Chlormezanon, Memantin, Tizanidin
Narkosemittel
   Methohexital, Propofol, Etomidat, Ketamin, Alfentanil, Thiopental, Droperidol, Fentanyl, Alfentanil, Sufentanil
Nebenschilddrüsenhormone, Calciumstoffwechselregulatoren
   Dihydrotachysterol, Calcitonin, Clodronsäure, Etidronsäure, Pamidronsäure
Neuropathiepräparate
   α-Liponsäure
Prostaglandine
   Alprostadil
Psychopharmaka
   Benzodiazepine (Lorazepam, Diazepam), Clomethiazol,
Schilddrüsentherapeutika
   1-Thyroxin, Carbimazol, Thiamazol, Propylthiouracil
Sera, Immunglobuline, Impfstoffe
   a) Immunglobuline allgemein und spezifisch wie Hepatitis-Typen, Röteln, Cytomegalie, Tollwut, FSME, Varicella-Zoster, Tetanus, Rhesusfaktoren
   b) Immunsera wie Botulismus-Antitoxin, Diphterie, Gasbrand, Schlangengift, Skorpiongift
   c) Impfstoffe wie Influenza, Tuberkulose, Cholera, Diphterie, Hepatitis-Typen, FSME, Röteln, Hämophilus influenzae, Masern, Neisseria, Mumps, Poliomyelitis, Tetanus, Tollwut, Typhus
Sexualhormone und ihre Hemmstoffe
   Anabolika, Androgene, Antiandrogene, Gestagene, Estrogene, Antiestrogene (Tamoxifen etc.), Flutamid, Fosfestrol, Cyproteron, Formestan, Aminoglutethimid
Toxoplasmosemittel
   Atovaquon
Urologika
   Trospiumchlorid
Vitamine
   Alfacalcidol, Vitamin A und -derivate, Vitamin E und -derivate, Ascorbinsäure
ZNS-Therapeutika
   a) Neuroleptika wie Perazin, Promazin, Sulpirid, Thioridazin, Chlorprothixen, Levomepromazin, Prothipendyl, Chlorpromazin, Clopenthixol, Triflupromazin, Perphenazin, Trifluperazin, Pimozid, Reserpin, Fluphenazin, Haloperidol, Trifluperidol, Benperidol, Alimemazin, Fluphenazin, Flupentixol, Melperon, Bromperidol, Pipamperon, Clozapin, Risperidon,
   b) Antidepressiva wie Imipramin, Desipramin, Trimipramin, Lofepramin, Clomipramin, Opipramol, Amitriptylin, Amitriptylinoxid, Nortriptylin, Dibenzepin, Doxepin, Maprotilin, Mianserin, Fluoxetin, Fluvoxamin, Paroxetin, Trazodon, Moclobemid, Tranylcypromin, Oxitriptan, Viloxazin, Hypericin, Lithiumsalze
   c) Tranquillantien wie Meprobamat, Hydroxyzin, Benzodiazepine wie Chlordiazepoxid, Diazepam, Prazepam, Oxazepam, Dikalium-clorazepat, Lorazepam, Clonazepam, Bromazepam, Clotiazepam, Alprazolam, Clobazam, Buspiron
   d) Psychostimulantien wie Coffein, Theophyllin, Theobromin, Amphetamine und verwandte Substantzen
   e) Stoffe zur Behandlung dementieller Syndrome wie Meclofenoxat, Nicergolin, Piracetam, Pyritinol, Tacrin, Memantin, Dihydroergotoxinmethansulfonat
   f) Appetitzügler wie Nor-pseudoephedrin, Amfepramon, Mefenorex, Levopropylhexedrin, Fenfluramin, Dexfenfluramin
   g) Analeptika wie Doxapram, Fenetyllin
   h) Nervenwachstumsfaktor (Nerve growth factor), Naloxon, Dalargin
   i) Antiparkinsonmittel wie L-Dopa, Selegilin, Bromocriptin, Amantadin, Tiaprid, Biperiden, Trihexylphenidyl, Procyclidin, Benzatropin, Orphenadrin, Bornaprin, Metixen, α-Dihydroergocryptin, Carbidopa
Zystostatika und Metastasenhemmer
   a) Alkylantien wie Nimustin, Melphalan, Carmustin, Lomustin, Cyclophosphamid, Ifosfamid, Trofosfamid, Chlorambucil, Busulfan, Treosulfan, Prednimustin, Thiotepa,
   b) Antimetabolite wie Cytarabin, Fluorouracil, Methotrexat, Mercaptopurin, Tioguanin
   c) Alkaloide wie Vinblastin, Vincristin, Vindesin
   d) Antibiotika wie Aclarubicin, Bleomycin, Dactinomycin, Daunorubicin, Doxorubicin, Epirubicin, Idarubicin, Mitomycin, Plicamycin
   e) Komplexe von Nebengruppenelementen (z.B. Ti, Zr, V, Nb, Ta, Mo, W, Ru, Pt) wie Carboplatin, Cisplatin und Metallocenverbindungen wie Titanocendichlorid
   f) Amsacrin, Dacarbazin, Estramustin, Etoposid, Hydroxycarbamid, Mitoxanthron, Procarbazin, Temiposid
   g) Alkylamidophospholipide (beschrieben in J.M. Zeidler, F. Emling, W. Zimmermann und Roth, H. J., Archiv der Pharmazie, 324, 687, 1991)
   h) Etherlipide wie Hexadecylphosphocholin, Ilmofosin und Analoga, (beschrieben in Zeisig, R., Arndt, D., Brachwitz, H., Pharmazie 45 809-818 1990)
   i) Taxane wie Paclitaxel und Docetaxel
   j) Altretamin, Aminoglutethimid, Asparaginase, Hydroxycarbamid, Miltefosin

### Beispiele

### Beispiel 1:

Unmodifizierte, Dalargin-beladene PBCA-Nanopartikel waren nicht in der Lage, nach i.v. Applikation die Blut-Hirn-Schranke zu überwinden. Die i.v. Applikation dieser Nanopartikel mit präadsorbiertem ApoE an Mäuse führte jedoch in dem von Alyautdin et al. beschriebenen "tail-flick-Test" (Alyautdin, R. N., Gothier, D., Petrov, V. E., Kharkevich, D. A., Kreuter, J., *Eur. J. Pharm. Biopharm*., 41, 44-48, 1995) zu einer analgetische Wirkung. 15 Minuten nach Injektion der Partikel wurde ein Effekt beobachtet, der 44% des maximal möglichen Effektes ausmachte (Berechnung des Effektes nach einer von Alyautdin et al. (Alyautdin, R. N., Gothier, D., Petrov, V. E., Kharkevich, D. A., Kreuter, J., *Eur. J. Pharm. Biopharm*., 41, 44-48, 1995) angegebenen Formel).

### Beispiel 2:

Es wurden ApoE-Lösungen (50 mg ApoE in 160 µl NH₄HCO₃-Puffer (10 mM), pH 7,5, Calbiochem-Novabiochem, Nottingham, UK) in jeweils 500 µl einer 6prozentigen Suspension (m/V) von unmodifizierten und mit Tween® 80 modifizierten PBCA-Partikeln für 3 h bei 37°C (Tamada und Ikada, 1993) inkubiert. Die Partikel wurden durch Zentrifugation vom Dispersionsmedium separiert und viermal gewaschen. Das adsorbierte ApoE wurde mit von Hochstrasser et al. (Hochstrasse, D. F., Harrington, M. G., Hochstrasser, A.-C., Miller, M. J., Merril, C. R., Anal. Biochem., 173, 424-435, 1988) beschriebenen solubilisierenden Lösungen von der Partikeloberfläche desorbiert. Jeweils 80 µl der proteinhaltigen Lösungen wurden auf die Röhrchengele der 1. Dimension der 2-DE appliziert. Der Nachweis des adsorbierten ApoE erfolgte mit zweidimensionaler Elektrophorese (2-DE) nach Blunk (Blunk, T., Hochstrasser, D. F., Sanchez, J.-C., Müller, B. W., Müller, R. H., *Electrophoresis,* 14, 1382-1387, 1994).

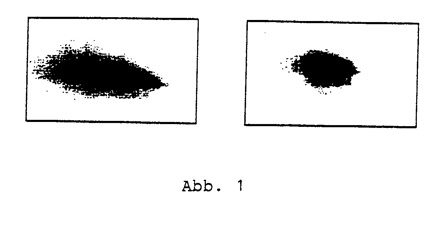

Abb. 1 zeigt die resultierenden ApoE-Spots auf den 2-DE-Gelen der unmodifizierten und mit Tween® 80 modifizierten PBCA-Partikel. Die Anreicherung von ApoE ist dabei auf den unmodifizierten Partileln am höchsten.

### Beispiel 3:

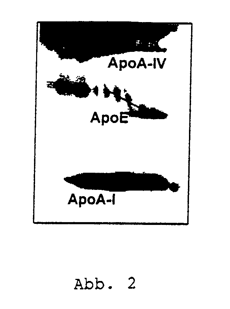

Um zu zeigen, daß andere Proteine das auf den PBCA-Partikeln präadsorbierte ApoE nach einer i.v. Applikation von der Partikeloberfläche nicht vollständig verdrängen, wurden die Partikel mit präadsorbiertem ApoE (vgl. Beispiel 2) gemäß dem Standardprotokoll für die 2-DE in Plasma inkubiert (5 min bei 37°C, nach Blunk, siehe Beispiel 2) und die resultierenden Adsorptionsmuster bestimmt. Abb. 2 zeigt Ausschnitte aus dem 2-DE-Gel, die die ApoE-Spots enthalten.

### Beispiel 4:

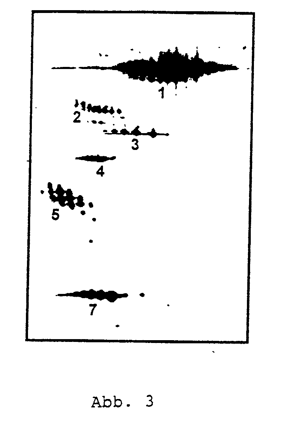

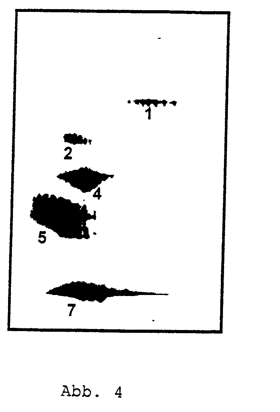

Es wurden die Plasmaproteinadsorptionsmuster von PBCA-Partikeln bestimmt, die keine analgetische Wirkung von Darlagin im ZNS vermittelten. Abb. 3 zeigt das mit unmodifizierten PBCA-Partikeln erhaltene Gel. Es wurde kein ApoE detektiert. Abb. 4 zeigt das mit Poloxamer 407 modifizierten PBCA-Partikeln erhaltene Gel. Es wurde ebenfalls kein ApoE detektiert und somit gezeigt, daß es in Abwesenheit von ApoE zu keiner ZNS-Wirksamkeit kommt.

### Beispiel 5

Chemische Kopplung von Apolipoprotein E an carboxylierte Polymethyl-methacrylat-Nanopartikel:

Ein mit Carboxylgruppen auf der Oberfläche funktionalisierter Polymethylmethacrylat-Latex (Partikeldurchmesser 65nm ± 10%) wird nach 2 Waschschritten (30.000 rpm / 10 min, 4°C) mit 0.01M Phosphat-Pufferlösung pH 6.5 auf 0.5% eingestellt. Bei 4°C werden 0.5 ml des Latex mit 300µl Proteinlösung versetzt und 60 min inkubiert. Jetzt erfolgt die Zugabe von 10mg wasserlöslichem Carbodiimid(1-Ethyl-3-(3-dimethylaminopropyl)-carbodiimidhydrochlorid). Das Gemisch wird über Nacht (16h) auf einem Schüttler mit niedriger Frequenz bewegt. Durch Zentrifugation (30.000 rpm / 10 min, 4°C) werden die Partikel vom Medium getrennt und in 0.1 M Glyzinpuffer pH 8.55 zu einem Feststoffgehalt von 0.5% aufgenommen.

### Beispiel 6

Chemische Kopplung von Apolipoprotein E an durch Einführung von Epoxy-Grupen oberflächenfunktionalisierten Polymethylmethacrylat-Latex:

Ein Kern-Schale-Latex (K: Polymethylmethacrylat; S: Polyglycidylmethacrylat) mit einem Partikeldurchmesser von 70nm ± 10% wird nach 2 Waschschritten (30.000 rpm / 10 min, 4°C) mit 0.0025M Phosphatpuffer pH 8.0 auf 2% Feststoffgehalt eingestellt. Gleiche Volumina des Latex und einer 0.2% igen Proteinlösung werden für 3h bei 28°C auf einem Schüttler bewegt. Anschließend wird zentrifugiert (30.000 rpm / 10 min, 4°C) und das Sediment in GBS-Puffer pH 8.0 aufgenommen, so daß der Latex einen Feststoffgehalt von 0.5% aufweist.

**Tabelle 1:**

| ZNS-Wirksamkeit von unmodifizierten und mit verschiedenen Tensiden oberflächenmodifizierten, mit Darlagin beladenen PBCA-Nanopartikeln nach intravenöser Gabe an Mäuse (Dalargin-Dosis: 10 mg/kg). Angegeben sind die analgetischen Effekte ausgedrückt als Prozentsatz vom maximal möglichen Effekt (% MME) (S: Standardabweichung, n=4) (Berechnungsformel nach Alyautdin, R. N., Gothier, D., Petrov, V. E., Kharkevich, D. A., Kreuter, J., *Eur. J. Pharm. Biopharm*., 41, 44-48, 1995) | | | | |
|---|---|---|---|---|
| **oberflächen- modifizierendes Tensid** | **% MME (nach 15 min)** | **S %** | **MME (nach 45 min)** | **S** |
| Tween 20 | 79,7 | ± 21,3 | 52,9 | ± 20,9 |
| **Tween 40** | 87,5 | ± 16,1 | 60,8 | ± 38,0 |
| **Tween 60*** | -7,1 | ± 24,2 | 45,5 | ± 36,6 |
| **Tween 80** | 100 | ± 0 | 10,5 | ± 14,9 |
| **Poloxamer 407** | 4,4 | ± 3,9 | 9,5 | ± 5,8 |
| **Poloxamer 908** | -1,3 | ± 3,6 | 4,2 | ± 5,5 |
| **Poloxamer 188** | 8,1 | ± 5,9 | 3,3 | ± 3,4 |
| **Poloxamer 184** | 0,9 | ± 0,28 | 1,0 | ± 2,3 |
| **Poloxamer 338** | 0,2 | ± 0,5 | 1,4 | ± 3,9 |
| **Cremophor EL** | 10,9 | ± 13,1 | 8,6 | ± 8,7 |
| **unmodifizierte Par- tikel** | 2,3 | ± 1,6 | 3,7 | ± 11,7 |

Dalargin-Dosis: 7,5mg/kg
**Abb. 1:** Ausschnitte mit ApoE-Spots aus 2-DE-Gelen von unmodifizierten PBCA-Partikeln (links) und von mit Tween 80 modifizierten PBCA-Partikeln (rechts) jeweils nach Präadsorption von ApoE. Durch das adsorbierte ApoE besaßen auch die unmodifizierten Partikel eine ZNS-Wirksamkeit
   (Beispiel 2).
**Abb. 2:** Ausschnitt aus dem 2-DE-Gel von unmodifizierten PBCA-Partikeln mit präadsorbiertem ApoE nach Inkubation in Plasma (Beispiel 3).
   Abszisse: nicht linearer Gradient pI 4,5 - 6,0
   Ordinate: nicht linearer Gradient MG 25.000 - 46.000
**Abb. 3:** 2-DE-Gel von unmodifizierten PBCA-Partikeln ohne ZNS-Wirksamkeit (Beispiel 4).
   (1) Albumin, (2) α1-Antitrypsin, (3) Fibrinogen γ, (4) ApoA-IV, (5) ApoJ, (7) ApoA-I
   Abszisse: nicht linearer Gradient pI 4,5 - 6,5
   Ordinate: nicht linearer Gradient MG 25.000 - 75.000
**Abb. 4:** 2-DE-Gel von mit Poloxamer 407 modifizierten PBCA-Partikeln ohne ZNS-Wirksamkeit (Beispiel 4)
   (1) Albumin, (2) α1-Antitrypsin, (4) ApoA-IV, (5) ApoJ, (7) ApoA-I
   Abszisse: nicht linearer Gradient pI 4,5 - 6,5 Ordinate: nicht linearer Gradient MG 25.000 - 75.000

## Patentansprüche

1. Arzneistoffträgerpartikel, in Wirkstoff-beladener oder Wirkstoff-freier Form, **dadurch gekennzeichnet, daß** an die Partikeloberfläche mindestens ein Erkennungsprotein oder zumindest ein Rezeptor-erkennender Molekülteil davon gebunden ist.

2. Arzneistoffträgerpartikel nach Anspruch 1, **dadurch gekennzeichnet, daß** das Partikelmaterial Polymere, den Arzneistoff (Nanosuspensionen, Hydrosole), feste Lipide, flüssige Lipide, o/w-Emulsionen, w/o/w-Emulsionen oder Phospholipidvesikel umfaßt.

3. Arzneistoffträgerpartikel nach Anspruch 1 oder 2, **dadurch gekennzeichnet, daß** das Erkennungsprotein durch unspezifische oder spezifische Adsorption an die Oberfläche der Partikel gebunden ist.

4. Arzneistoffträgerpartikel nach Anspruch 1 oder 2, **dadurch gekennzeichnet, daß** das Erkennungsprotein kovalent an die Oberfläche der Partikel gebunden ist.

5. Arzneistoffträgerpartikel nach Anspruch 4, **dadurch gekennzeichnet, daß** das die Bindung an Partikel mit reaktiven Oberflächengruppen, insbesondere Epoxy- oder Aldehydgruppen, oder nach Aktivierung der Partikeloberfläche mit Aktivatoren, insbesondere Carbodiimid, N-Eth-oxycarbonyl-2-ethoxy-1,2-dihydrochinolin, Glutardialdehyd, Bromzyan, meta-Perjodat (Na-Salz oder K-Salz), Tosylchlorid und Chlorameisensäureester, bewirkt worden ist.

6. Arzneistoffträgerpartikel nach Anspruch 5, **dadurch gekennzeichnet, daß** das die Bindung der Erkennungsproteine über ihre Aminogruppen bewirkt worden ist.

7. Arzneistoffträgerpartikel nach Anspruch 1 oder 2, **dadurch gekennzeichnet, daß** das Erkennungsprotein durch präferentielle Adsorption an die Oberfläche der Partikel gebunden ist.

8. Arzneistoffträgerpartikel nach Anspruch 7, **dadurch gekennzeichnet, daß** die präferentielle Adsorption aus Proteinlösungen oder durch Kontakt der Partikel mit Plasma, Serum oder Blut erfolgt, wobei letzteres auch ex vivo oder in vivo erfolgen kann.

9. Arzneistoffträgerpartikel nach Anspruch 7 oder 8, **dadurch gekennzeichnet, daß** die Oberfläche der Partikel vor der präferentiellen Adsorption durch Einführung funktioneller Gruppen, insbesondere Hydroxyl-, Carboxyl-, Amino-, Hydroxyethyl, Epoxy oder Aldehydgruppen und deren Derivate chemisch modifiziert wurde oder durch physikalische Behandlung mit Plasma, insbesondere plasma etching, zur Einführung von Hydroxylgruppen in den Eigenschaften verändert wurde.

10. Arzneistoffträgerpartikel nach Anspruch 7, 8 oder 9, **dadurch gekennzeichnet, daß** die Oberfläche durch Adsorption von Substanzen modifiziert wurde, die zu einer präferentiellen Adsorption des Erkennungsproteins führen, wobei die modifizierende Substanz im Verhältnis zum Arzneistoffpartikel in einer gewichtsbezogenen Menge von 0.01 Teile bis 10 modifizierende Susbstanz pro 1 Teil Partikel, vorzugsweise 0,1 bis 10 Teile modifizierende Susbstanz pro 1 Teil Partikel, und insbesondere 1 Teil modifizierende Susbstanz pro 1 Teil Partikel eingesetzt wird.

11. Arzneistoffträgerpartikel nach Anspruch 10, **dadurch gekennzeichnet, daß** ihre Oberfläche durch die Adsorption von Tensiden, insbesondere ethoxylierten Tensiden, vorzugsweise Polyethylenglykol-Fettsäureestern und Polyethylenglykol-Fettalkoholethern, bevorzugt Polyetylenglykol-Sorbitanfettsäureestern und Polyethylenglykol-Fettsäureglyceriden, bevorzugter Tween® 20, 40, 60 und 80 oder Cremophor® EL und RH40 modifiziert worden ist.

12. Arzneistoffträgerpartikel nach Anspruch 10, **dadurch gekennzeichnet, daß** ihre Oberfläche durch die Adsorption von Polymeren, insbesondere Polymeren aus der Poloxameren und Poloxaminen, Cellulosen und ihren Derivate, vorzugsweise Methylcellulose, Hydroxyethylcellulose, Hydroxypropyl-Methylcellulose, Hydroxypropylcellulose, Carboxymethylcellulose-Natrium sowie Xanthan, Polyvinylalkohol, Polyvinylpyrrolidon, Polyacrylsäuren, Polyethylenglykolen, Polyethylenglykol-enthaltenden Block-Copolymere, Stärke und -derivaten, Dextran und -derivaten, Polyethylenimin und Gelatine modifiziert worden ist.

13. Arzneistoffträgerpartikel nach einem der vorhergehenden Ansprüche, **dadurch gekennzeichnet, daß** das Erkennungsprotein ein im Blut natürlich vorkommendes Erkennungsprotein ist.

14. Arzneistoffträgerpartikel nach einem der vorhergehenden Ansprüche, **dadurch gekennzeichnet, daß** das Erkennungsprotein Apolipoprotein E, Apolipoprotein A-I, A-II, A-IV, B, C-II, C-III, D, H und/oder J ist.

15. Arzneistoffträgerpartikel nach Anspruch 14, **dadurch gekennzeichnet, daß** das Erkennungsprotein Apolipoprotein E in Kombination mit einem oder mehreren anderen Erkennungsproteinen vorliegt, insbesondere mit Apolipoprotein A-I, A-II, A-IV, B, C-II, C-III, D, H und/oder J, und/oder mit Albumin.

16. Arzneistoffträgerpartikel nach einem der vorhergehenden Ansprüche, **dadurch gekennzeichnet, daß** Apo C-II, Apo C-III, Apo A-IV, Apo-E einzeln oder in Kombinationen von 2 oder 3 oder 4 Apolipoproteinen eingesetzt werden.

17. Arzneistoffträgerpartikel nach einem der vorhergehenden Ansprüche, **dadurch gekennzeichnet, daß** Erkennungsprotein, bezogen auf den Arzneistoffträger, in einer Menge von 0,001 bis 40 Gew.-%, insbesondere von 0,01 bis 30 Gew.-% und bevorzugt von 0,1 bis 15 Gew.-% vorhanden ist.

18. Arzneistoffträgerpartikel nach Anspruch 17 zur verlängerten Zirkulationszeit im Blut, die eine Menge an ApoE enthalten, die für eine anreicherung im ZNS nicht goß genug ist, oder kein ApoE in Kombination mit einem oder mehreren der Apoliporoteine A-I, A-II, A-IV, C-II und C-III umfassen.

19. Arzneistoffträgerpartikel nach einem der vorhergehenden Ansprüche, **dadurch gekennzeichnet, daß** sie in Form von festen oder flüssigen Teilchen, von Suspensionen oder Emulsionen fester oder flüssiger Teilchen vorliegen, die vorzugsweise amorphe oder kristalline Nano- oder Mikropartikel, o/w-Emulsionen, w/o/w-Emulsionen oder Liposomen umfassen, oder von Nonosuspensionen oder Hydrosolen vorliegen.

20. Verwendung von Arzneistoffträgerpartikeln gemäß einem der vorhergehenden Ansprüche zur Herstellung von Arzneimitteln.

21. Verwendung von Arzneistoffträgerpartikeln nach Anspruch 20, **dadurch gekennzeichnet**, das die Arzneimittel im zentralen Nervensystem anreicherbar sind.

22. Verwendung nach Anspruch 20, **dadurch gekennzeichnet**, das die Arzneimittel in Geweben, an Zielorten oder in Zellen, insbesondere im Knochenmark, in der Leber, in der Milz, in Tumorgeweben, in Tumormetastasen sowie in sämtlichen Zellen des Blutes - kernkaltig und kernlos anreicherbar sind.

23. Verwendung des Arzneistoffträgerpartikels nach Anspruch 20, **dadurch gekennzeichnet**, das die Arzneimittel über einen längeren Zeitraum im Blut zirkulierbar sind.

24. Verwendung von Erkennungsproteinen oder Rezeptor-erkennenden Molekülteilen davon zur Herstellung von Arzneimitteln bei der sie an die Oberfläche der jeweiligen Arzneimittelträger gebunden werden.

25. Verwendung von wirkstoff-freien oder wirkstoff-beladenen Partikeln zur Bindung an mindestens ein Erkennungsproteinen oder einen Rezeptor-erkennenden Molekülteile davon zur Herstellung von Arzneimitteln, wobei die Bindung an der Oberfläche der jeweiligen Partikel erfolgt.
